Europäisches Patentamt

European Patent Office

Office européen des brevets

(11) Publication number: **0 150 712**
A2

(12) # EUROPEAN PATENT APPLICATION

(21) Application number: **85100061.2**

(51) Int. Cl.⁴: **C 07 G 3/00**

(22) Date of filing: **03.01.85**

(30) Priority: **04.01.84 ES 528692**

(43) Date of publication of application: **07.08.85**
**Bulletin 85/32**

(84) Designated Contracting States: **AT BE CH DE FR GB IT LI NL SE**

(71) Applicant: **Bioiberica, S.A., Poligono Industrial s/n, Palafolls Barcelona (ES)**

(72) Inventor: **David Nusimovich, Alberto, Residencial Port-Mar s/n, San Andres de Llavaneras (Barcelona) (ES)**
Inventor: **Vila Pahl, Francisco Javier, Avenida Pearson, 115-117, ES-08034 Barcelona (ES)**

(74) Representative: **Dipl.-Ing. Schwabe, Dr. Dr. Sandmair, Dr. Marx, Stuntzstrasse 16, D-8000 München 80 (DE)**

(54) **Method for the obtention of a glycosphingolipid complex.**

(57) The invention relates to a process for the obtention of a glycosphingolipids complex characterized by the preparation in the first step of an acetone-dried powder of animal brains by means of their anhydrification through successive acetone treatments; then, from the prepared powder, gangliosides are axtracted with a solvent solution (water/methanol/chloroform) in a way that they remain dissolved in the aqueous phase impurified with phospholipids; then a purification is performed by Silica gel columm chromatography changing the polarity of eluent from an apolar solvent mixture that eluates phospholipids, remaining the gangliosides in the columm, to a more polar one that eluates gangliosides. Once the gangliosides are obtained, these are fractionated through ionic-exchange columm chromatography. Finally, ganglioside mixture is reformulated in order to obtain the desired glycosphingolipid complex.

Procedure for the obtention of a glycosphingolipid complex:

## DESCRIPTION

The present invention relates to a method of preparing a new glycosphingolipid complex obtained from mammalian brains through an extractive procedure.

Glycosphingolipids are substances composed of sphingosine, fatty acid and carbohydrate. They are all derivatives of ceramide (N-acylsphingosine) that is bound to carbohydrate groups via OH at carbon atom 1 in the sphingosine. The glycosphingolipids has been classified in three groups according to their structure:

a) Neutral glycosphingolipids, which are composed of a ceramide bound to a glycosyl unit (cerebroside) or to an oligoglycosylceramide; b) sulfatides; c) gangliosides, the most complex group, with their polysacharides bound to one or more sialic acid residues.

Structural study of gangliosides has been difficult due to the fact that they appear as minor components in the animal tissues and also for their complex chemical structures and physicochemical properties.

Therefore, isolation of significant amounts of that substances, involves the extraction of large quantities of animal tissues, followed by a complex purification through different chromatographic methods.

Different studies on the biological role of glycosphingolips suggest their efficacy as receptors for bacterial neurotoxins (cholera, tetanus, etc ). Ever since their discovery they have received particular attention for their involvement in the functions of the brain like nerve conduction (supported by their high occurrence in the synaptosomes) and

0150712

for their inmunological properties as they are partially responsibles of Lewis and ABO human blood groups. The specificuess of A, B and H antigens are determined by the reducing structures of glycosphingolipids oligosacharides.

In the present description, nomenclature is based in the system proposed by Svemmerholm (1963-1970). The G of ganglioside is followed by the letter M, D or T which designs if the compound is mo, di or trisialoganglioside and with a number 5-n where n is the number of neutral carbohydrate residues.

According to the present invention the procedure for the obtention of the glycosphingolipid complex is essentially characterized by the following operations:

a) Preparation of acetona-dried powder of brains

It consists in an anhydification of brain tissues by succesive treatments with acetone.

b) Obtention of crude gangliosides

The process is basically an extraction of gangliosides from the acetone-dried powder prepared in a), performed by a mixture of water-methanol-chloroform. In this way impure gangliosides are partitioned in the upper water-methanol layer and phospholipids remain in the lower chloroform-methanol layer.

c) Purification of gangliosides

Purification is achieved by the use of silica-gel chromatography changing the polarity of eluent. Phospholipids are eluted with an apolar eluent remaining the gangliosides in the silica-column.
Then, the gangliosides are eluted using more polar eluent.

d) Separation in fractions from the ganglioside mixture

Once obtained a ganglioside mixture with a title not less than 275 mg NANA/g, these are fractionated by ionic-exchange chromatography using as eluent an ionic solution with a linear gradient concentration.

e) Recomposition of a percentage fromulation of gangliosides

Following is an illustrative but not limitative example of the procedure that is subject of the present patent of invention.

Example

a) Preparation of acetone-dried powder of brains

200 1. of 95% acetone are added 50 kg of bovine brains, the mixture is agitated during 15-20 min. The mixture is allowed to decant; supernatant is eliminated and 150 1. of 95% acetone are added to the residual paste following with 15-20 min. of agitation. The extraction is repeated several times until a 5-10% moisture content is attained in the paste. Paste is sieved to segregate fibrous matter, filtered and dried in a vacuum-oven at 30°C and 30 mm Hg pressure. 8.0 kg of powder (SI) are obtained with a title of 5.5 mg NANA determined with the analytical method (TA II).

b) Obtention of crude gangliosides

30 1. of water are added to 8 kg. of acetone-dried powder (SI), the mixture is agitated during 25-30 min. and then 80 1. of methanol and 40 1. of Chloroform are added. The agitation is mantained during 15 hours, at room temperature in a closed vessel. Then is filtered and the liquid (LI) is left.

Residual paste is resuspended in a mixture of: 40 1. methanol, 20 1. chloroform and 15 1. water and agitated during 6 hours. Then is filtered and filtered solution (LII) is combined with liquid (LI) obtaining a solution with a title of 20.2 mg NANA/g determined with the analytical method TAII. To this solution 30 1. water are added and the mixture is decanted overnight in a closed vessel obtaining two liquid phases: an aqueous phase (LIII) and a chloroformic one (LIV).

The chloroform amount of the aqueous phase (LIII) that contains the gangliosides (21.0 mg NANA/g), determined with the analytical method (TAII), is removed by distillation under vacuum at 40°C and 160 mm Hg presure.

The resulting turbid solution is clarified through membrane filters. It is obtained a clarfied solution (LV) with a title of 42,5 mg NANA/g determined with the analytical method TAII.

The solution (LV) is reduced in volume six times by concentration and then dialyzed by ultrafiltration through a membrane of 10.000 daltons molecular weight cur-off, until total elimination of salts and pigments is obtained. The dialyzed solution (LVI) is lyophilized and 220 g of a solid (SII) are obtained with a title of 136 mg NANA/g, determined with the analytical method (TAII).

c) Purification of gangliosides

Silica gel 0.063-0.200 mm (70-230 mesh) is suspended in a mixture of chloroform-methanol-water (110:40:6, v/v) and the slurry is packed in a chromatrographic column 9.6 cm diameter, 96 cm height. A suspension of one part in weight of solid (SII) obtained in b) and ten parts in weight of adsorbent, in the mixture of solvents mentioned above, is applied to the column and then eluted with that eluent until the phospholipis are not detectable in the eluate by Thin-Layer Chromatography (approximately after elution with ten bed volumes of eluent).

Once this point has been reached, the polarity of eluent is changed and a mixture of chloroform-methanol- 0.25% aqueous Potassium Chloride solution (60-35:8 v/v) is passed through the column until no more gangliosides are detected in the eluates by the resorcinol reaction. In this way, the gangliosides, dissolved in the second eluent, are separated from phospholipids. The chloroform in ganglioside solution is removed by distillation at 40°C and 160 mm Hg pressure and the resulting solution is concentrated by Ultrafiltration through a membrane of 10.000 daltons M.W. cut-off. Once the volume has been reduced ten times, the resulting solution is lyophilized, obtaining 66 g of mixed pure gangliosides (SIII) with a title of 312 mg NANA/g determined with the analytical method (TAII).

   d) Separation in fractions from the ganglioside
      mixture.

   One volume of DEAE-Sephadex A-25 is thoroughly suspended in ten volumes of chloroform-methanol-0.8M Sodium acetate (30:60:8, v/v) with vigorous swirling, left for 30 min. and filtered. This procedure is repeated two times. The gel is then suspended in the the same solvent mixture and kept at 4°C overnight. After filtration, the gel is washed three times with 10 volumes of chloroform-methanol-water (30:60:8, v/v) and the slurry in the same solvent is introduced into a columm 9.6 cm diameter, 96 cm height.

      The sample of 66 g pure gangliosides (SIII) obtained in c) is dispersed in 800 ml of chloroform-methanol (1:1, v/v), mixed with 66 g DEAE-Sephadex A-25 (acetate form) during 20 min. The suspension is then diluted with 400 ml methanol and 100 ml water to give a final solvent mixture of chloroform-methanol-water (30:60:8, v/v) and applied to the columm. A first elution is performed with three bed-volumes of chloroform: methanol-water (30:60:8, v/v) followed by one bed volume of methanol. Gangliosides are then eluted with a linear gradient of ammonium acetate in methanol (0.05M, 0.15M, 0.45M) until negative reaction by the resorcinol assay is obtained in the collected fractions. According to the results obtained by thin-Layer chromatography on the eluted fractions, these are combined in three major fractions named LVII, LVIII and LIX.

Chloroform contained on each fraction is removed by distillation at 40°C, 160 mm pressure and resulting fractions are concentrated ten times by ultrafiltration with membrane of 10.000 dalton M.W cut-off. The three concentrates are lyophilized and then combined in order to obtain a solid (SIV) with the following composition:

| GANGLIOSIDE | PERCETAGE |
|---|---|
| $GM_1$ | 19-21 |
| $GD_{1a}-GD_{1b}$ | 53-63 |
| $GT_{1b}$ | 18-20 |

## CHEMICAL IDENTIFICATION

Thin-Layer Chromatography of Gangliosides

TA I:

Sample solution is prepared, dissolving 50 mg/ml in a mixture chloroform-methanol (2:1, v/v).

10 $\mu$l sample solution are applied on a chromatoplate of Silica-Gel 60 $F_{254}$ (Merch) developing with a mixture of chloroform-methanol-0,3% calcium chloride aq. solu. (16:11:3) during one hour and thirty min. The detection reagent is sprayed on the plate followed by heating in an oven at 120°C for 15 min.

Detection reagent: 200 mg resorcinol are dissolved in 10 ml water. 40 ml concentrated hydrochloric acid and 2 ml of 0.1M $CuSO_4$, $5H_2O$ aq. solu. are added to resorcinol solu. Total volume is brought to 100 ml with distilled water.

Gangliosides give blue spots. Identification of different compounds is performed running in paralled a standard prepared under the same conditions. Rf values of individual species increases in the order: $GT_{1b}$-$GD_{1b}$-$GD_{1a}$-$GM_1$ .

TA II: Determination of N-acetylneuraminic acid

Preparation of sample solution: 25 mg sample are exactly weighed and dissolved in 25 ml water.

Preparation of standard solution: 10 mg pure N-acetil neuraminic acid (Fluka) are accurately weighed and dissolved in water. Total volume is brought to 50 ml with water in a volumentric flask (0.2 mg/ml final conc.) '

Preparation of resorcinol reagent: 200 mg resorcinol are dissolved in 10 ml water. 80 ml concentrated hydrochloric acid and 0.25 ml of 0.1M $CuSO_4$.$5H_2O$ aq. solu. are added to resorcinol solution. Total volume is brought to 100 ml with water.

Standard curve: 0.02, 0.6 and 0.8 ml (0.40, 120 and 160 µg N-acetylneuraminic acid) of standard solution are pipetted into four tubes. The volume in each tube is made up to 2 ml with distilled*and 2 ml of resorcinol reagent are added to the four tubes. The tubes are heated for 20 min. at 90°C, and then inmmediately cooled. 5 ml of isoamyl alcohol are added, the tubes are shaken vigorously for 30 min. the supernatants are transferred to other tubes adding a small amount of anhidrous sodium sulfate to each tube. The extintion coefficient of the organic phase is read at 585 nm against the blank.

The standard absortion spectra is graphicated versus N-acetylneuraminic acid content of aliquots.

Sample solution: 0.4 ml of sample solution are pipetted into a 20 ml pyrex tube, the volume is brought to 2 ml with distilled water and 2 ml resorcinol reagent are added. It is followed the same procedure as for the standard

* water

solution and the extintion coefficient is read at 585 nm relating the obtained result with the standard curve previously graphicated.

## TA III: Identification of Sphingosine

<u>Reagent</u>: 120 mg vanillin are dissolved in 20 ml water and made up to 100 ml with 85% phosphoric acid.

<u>Method</u>: To 1 ml of ganglioside aqueous solution (1 mg/ml), 10 ml of reagent are added. The tubes are shaken and heated in a boiling water bath for 15 min. Then are cooled at room temperature and left during 30 min. A blue colour is developed.

## TA IV: Identification of carbohydrate mixture

<u>Reagent</u>: 50 mg anthrone and 1 g thiourea are dissolved in 100 ml of 66% sulfuric acid. The solution is heated for 15 min. at 80-90°C and then inmediately cooled at room temperature.

<u>Method</u>: 10 ml of reagent are added to 1 ml of ganglioside aqueous solution (1 mg/ml). The mixture is vigorously shaken and then heated for 15 min. in a boiling-water bath. It is cooled at room temperature and left for 30 min. A blue colour is developed.

## PURITY TESTS

## EP I: Thin-Layer chromatography of phospholipids

A 5 mg/ml sample solution is prepared, using as solvent mixture chloroform-methanol (2:1, v/v). 10 µl sample solution are applied on a chromatoplate of Silica-Gel 60 $F_{254}$ (Merck) developing with a mixture of chloroform-methanol-water (70:30:5, v/v). Lipids are detected as brown spoots with iodine vapor after 10-15 min. exposure.

EP II: Determination of Organic Phosphorus

<u>Reducing solution</u>: 0.1 g Tin are dissolved in 2 ml concentrated Hydrochloric acid. Volume is made up to 10 ml with distilled water (storage time: 3-4 days).

<u>Ammonium Molybdate Solution</u>: A 10% solution is prepared dissolving Ammonium Orthomolybdate in water, this solution is mixed with an equal volume of concentrated sulfuric acid.

<u>Preparation of standard</u>: 439.6 mg anhidrous monobasic potassium phosphate are dissolved in distilled water and volume is made up to 100 ml. 1 ml of this solution is pipetted to a 100 ml volumetric flask and volume is made up with distilled water. 1,2,3 and 5 ml of prepared solution are pipetted to each of four 50 ml volumetric flasks and final volumes are made up with distilled water. 2 ml ammonium molybdate solu. and 0.5 ml reducing solu. are added to each 50 ml solu. and left for 1 hour. The extintion coefficient is read at 525 nm against a blank.

<u>Test Solution</u>: 10 mg of exactly weighed gangliosides sample are dissolved in 10 ml $H_2O$/conc. $H_2SO_4$ (1:2,5). The mixture is carefylly heated and after charing , cooled at room temperature. A drop of 30% hydrogen peroxyde is added. Meanwhile shaking the mixture is heated at boiling temperature until a clear solution is obtained. Solution is cooled and 3 ml water are added and the solution is boiled for a while. The solution is cooled and the final volume is made up to 5 ml. The procedure is similar to the one carried on the standard. The extinction coefficient at 525 nm is read and the phosphorous percentage is estimated from the standard curve. The phosphorous content in the sample must be not more than 2.4 µg/ml equal to 60 µg phospholipids per mg of raw material.

EP III: <u>Free N-acetylneuraminic acid</u>

<u>Standard solution</u>: 50 µg/ml solution is prepared dissolving N-acetylneuraminic acid (Fluka) in distilled water.

<u>Test solution</u>: 20 µg sample are dissolved in 10 ml of distilled water (2 mg/ml).

<u>Reagents</u> :

1) 0.426 g Sodium Peryodate are dissolved in 3 ml distilled water, 6.1 ml of 85% Phosphoric acid are added and volume is made up to 10 ml with distilled water.

2) 10% w/v Sodium Arsenite in a solution of 0.5M Sodium Sulfate in 0.1N Sulfuric Acid.

3) 0.6% w/v thiobarbituric acid in 0,5M aq. Sodium Sulfate (must be freshly prepared).

4) <u>Cyclohexanone</u>

<u>Method</u>: 0.5 ml water and 0.2 ml of solution 1 are added to 0.1 ml standard solution. It is left for 20 min. at room temperature. 1 ml solution 2 (previously diluted with water, 1:10) are dropwise added. After shaking 3 ml solution 3 are added and the mixture is boiled for 15 min., then it is cooled and extracted with 4 ml of cyclohexanone.

0.5 ml test solution with 0.1 ml added water are assayed in parallel. Solution develops a pink colour. The absorbancy is read at 549 and 532 nm against a blank. Free N-acetylneuraminic acid in test-sample is estimated using the following equation:

$$\frac{(E_{549} \cdot 0.81 - E_{532} \cdot 0.32)_{std.}}{5 \ \mu g_{std.}} = \frac{(E_{549} \cdot 0.81 - E_{532} \cdot 0.32)_{sample}}{x \ \mu g_{sample}}$$

Free N-acetylneuraminic acid content must be less than 5 µg/ml.

EP IV: Aminoacids

Reagents: Ninhydrin (according 50 B.P. app. 1A A28.)

Method: 10 mg raw material are dissolved in 5 ml water. 0.5 ml from this solution are made up to 2 ml with water and 0.5 ml reagent are added. The solution is boiled for 15 min. Then it is cooled and diluted with 4 ml ethanol-water (1:1, v/v). The extintion coefficient is read at 500 nm against a blank.

A calibration curve is obtained using 0.2, 0.3 and 0.5 ml of a 40 µg/ml glutamic acid solution. The sample procedure is followed.

The aminoacids content expressed as glutamic acid must be less than 20 µg/ml.

EP V: Organic solvents

Organic solvents are non-detectable.

EP VI: Iron

Less than 20 ppm (FU VIII ed. Vol. 1, page 133)

In the practical performing of the subject of the present patent of invention, whatever detail could be changed that not affect its own essence.

0150712

## CLAIMS

It is claimed as the object af the present Patent of Invention:

1- Procedure for the obtention of a glycosphingolipids complex characterized by the preparation in the first step of an acetone-dried powder of animal brains by means of their anhydrification through successive acetone treatments; then, from the prepared powder, gangliosides are extracted with a solvent solution (water/methanol/chloroform) in a way that they remain dissolved in the aqueous phase impurified with phospholipids; then a purification is performed by Silica gel columm chromatography changing the polarity of eluent from an apolar solvent mixture that eluates phospholipids, remaining the gangliosides in the columm, to a more polar one that eluates gangliosides. Once the gangliosides are obtained, these are fractionated through ionic-exchange columm chromatography. Finally, ganglioside mixture is reformulated in order to obtain the desired glycosphingolipid complex.